# EUROPÄISCHE PATENTSCHRIFT

(11) **EP 1 189 626 B1**
(45) Veröffentlichungstag und Bekanntmachung des Hinweises auf die Patenterteilung: **08.10.2008**
(21) Anmeldenummer: 00938807.5
(22) Anmeldetag: 21.06.2000
(51) Int. Cl.: A61K 38/17, A61P 29/00, A61P 35/00, A61P 37/06

(54) **ZUSAMMENSETZUNG ZUR VERHINDERUNG VON TUMORWACHSTUM**
COMPOSITION FOR PREVENTING TUMORAL GROWTH
COMPOSITION PERMETTANT D'EVITER LA CROISSANCE TUMORALE

(30) Priorität: 28.06.1999 DE 19929488; 03.02.2000 DE 10004447
(43) Veröffentlichungstag der Anmeldung: 27.03.2002
(73) Patentinhaber: Abbott GmbH & Co. KG, 65205 Wiesbaden (DE)
(72) Erfinder: MÄNNEL, Daniela, N., D-93152 Schönhofen-Nittendorf (DE); HEHLGANS, Thomas, D-93173 Wenzenbach (DE); SEITZ, Carola, D-93051 Regensburg (DE)
(74) Vertreter: Grünecker, Kinkeldey, Stockmair & Schwanhäusser Anwaltssozietät
(86) Internationale Anmeldenummer: PCT/EP2000/005738
(87) Internationale Veröffentlichungsnummer: WO 2001/000228

(56) Entgegenhaltungen:
- WO-A-97/03687
- WO-A-98/17313
- WO-A-99/38525
- BROWNING JEFFREY L ET AL: "Signaling through the lymphotoxin beta receptor induces the death of some adenocarcinoma tumor lines." JOURNAL OF EXPERIMENTAL MEDICINE, Bd. 183, Nr. 3, 1996, Seiten 867-878, XP000971012 ISSN: 0022-1007 in der Anmeldung erwähnt
- MURTHY S ET AL: "LYMPHOTOXIN BETA RECEPTOR-IMMUNOGLOBULIN FUSION PROTEIN (LTBRFT) AMELIORATES INFLAMMATION AND TISSUE DAMAGE IN THE DEXTRAIN SULFATE (DSS) MODEL OF MOUSE COLITIS" GASTROENTEROLOGY,US,ELSEVIER, NEW YORK, NY,, Bd. 114, April 1998 (1998-04), Seite A1047 XP000915171 ISSN: 0016-5085
- HEHLGANS T ET AL: "Lymphotoxin-beta receptor activation promotes tumor growth by inducing angiogenesis." SCANDINAVIAN JOURNAL OF IMMUNOLOGY, Bd. 51, Nr. Supplement 1, Juni 2000 (2000-06), Seite 39 XP000971013 8th International TNF Congress, Conference on Tumor Necrosis Factor and Related Molecules Scientific Advances and Medical Applications;Trondheim, Norway; May 14-18, 2000 ISSN: 0300-9475

## Beschreibung

Die vorliegende Erfindung betrifft ein Arzneimittel zur Verhinderung von Tumorwachstum, indem man die Aktivierung, die über den Lymphotoxin β Rezeptor (LTBR) vermittelt wird, unterdrückt.

Das Membranprotein Lymphotoxin β ( Browning et al.,Cell Vol.72, 847-856, 1993) bindet in homomerer und heteromerer Form an einen spezifischen Lymphotoxin β Rezeptor (LTBR) (Crowe et al., Science Vol 264, 29 April 1994, 707-709).

Browning et al (J. Exp. Med. Vol. 183, March 1996, 867-878) beschreiben, daß die Signalvermittlung durch das LTBR-System den Zelltod für einige Adenocarcinoma Tumorzellen verursacht. Daher schlagen sie die LTBR-Aktivierung als mögliche Antikrebstherapie vor.

Degli-Esposti et al. (Journal of Immunology, 1997, 158: 1756-1762) berichten, daß Melanomzellen in ihrem Wachstum gehemmt werden können, wenn das LTBR-System aktiviert wird. Daher schlagen auch sie die Behandlung von soliden Tumoren mit Lymphotoxin β vor.

Demgegenüber zeigen unsere Befunde, daß sich Tumorwachstum verlangsamen oder verhindern läßt, indem man die LTBR-Aktivierung unterdrückt.

Gegenstand der Erfindung ist daher ein Arzneimittel zur Verhinderung von Tumorwachstum, indem man die LTBR-vermittelte Aktivierung unterdrückt oder die Aktivierung zumindest reduziert.

Das Arzneimittel ist besonders geeignet bei soliden Tumoren.

Die Unterdrückung des LTBR-Systems kann auf verschiedenen Ebenen erfolgen.

Beispielsweise kann die LTBR-Genexpression unterdrückt werden, indem man antisense Moleküle verwendet. Eine weitere Möglichkeit, die LTBR-Genexpression spezifisch zu unterdrücken, ist die mRNA des LTBR zu eliminieren, beispielsweise durch spezifische Hydrolyse mittels geeigneter Ribozyme.

Eine weitere Möglichkeit, die LTBR-Genexpression zu unterdrücken, ist die Blockade der entsprechenden Transkriptionssignalsequenzen, beispielsweise des Promotorbereichs des LTBR-Gens.

Eine andere Möglichkeit, die Aktivierung des LTBR-Systems zu unterdrücken, ist die Verwendung von Lymphotoxin β-Antagonisten, d.h. von Molekülen, die an den LTBR binden können, jedoch keine Aktivierung des Rezeptorsystems bewirken können. Solche Substanzen können aufgefunden werden, indem man die zu testenden Substanzen in einem zellulären Testsystem auf ihre Fähigkeit überprüft, die Aktivierung des LTBR-Systems in Gegenwart von zugesetztem Lymphotoxin β herabzusetzen.

Für die vorliegende Erfindung, wird ein Fusionsprotein aus LTBR und Immunoglobulin verwendet.

Ein weiterer Gegenstand der Erfindung ist die Verwendung von Substanzen, die die LTBR-vermittelte Aktivierung unterdrücken zur Herstellung von cytostatischen Arzneimitteln.

Es wurde beobachtet, daß die Unterdrückung der LTBR-Aktivierung eine Hemmung der Tumorangiogenese bewirkt und somit einen hemmenden Einfluß auf das Wachstum von soliden Tumoren hat.

Die Verhinderung der LTBR-Aktivierung mit den oben ausgeführten Mitteln und Verfahren eignet sich daher insbesondere zur Hemmung der Tumorangiogenese.

Die folgenden Experimente erläutern die Erfindung.

### Experiment 1

Tumorzellen (BFS-1) wurden mit einem Fusionsprotein aus LTBR und Immunglobulin transfiziert (BFS-LTR). Dieses Fusionsprotein aus LTBR und Immunglobulin agiert als LTBR-Inhibitor. Diese transfizierten Tumorzellen zeigten im Vergleich zu Wildtyp Tumorzellen ein gehemmtes Tumorwachstum, wenn sie in syngene Mäuse eingebracht wurden (Abb.).

### Experiment 2

Wurden in einem zweiten Experiment diese Tumorzellen in Liganddefiziente Mäuse eingebracht, (d.h. doppelt knock-out Mäuse, die weder Lymphotoxin alpha noch Lymphotoxin beta produzieren), ergaben sich keine signifikanten Unterschiede im Tumorwachstum der transfizierten gegenüber der Wildtyp Tumorzelle (Abb.).

### Experiment 3

### Intravitalmikroskopische Beobachtung des Tumorwachstums in Mäusen

Mäusen (C57BL/6) wurden normale Tumorzellen bzw. Tumorzellen, die mit dem LTβ-Rezeptor-Inhibitor (BSF-1-LTβR-Ig:Fc) oder mit einem Kontrollplasmid (p55TNF-Rezeptor-Ig-Fc) transfiziert waren, subkutan in einer Hautkammer appliziert und das Tumorzellwachstum bzw. die Angioneogenese intravitalmikroskopisch verfolgt. Schon die Ödembildung am Tag 2 nach Tumorzell-Applikation war bei den Tieren, die LTβ-Rezeptor-Inhibitor transfizierte Tumorzellen erhielten, deutlich geringer als in den Kontrollen. Während die normalen Tumorzellen und mit einem Kontrollkonstrukt transfizierte Tumorzellen nach 5 bis 9 Tagen deutliche Angiogenese induzierten und dementsprechend dann als Tumoren wuchsen, zeigte sich bei den Tumoren, die mit dem LTβ-Rezeptor-Inhibitor transfiziert sind, keinerlei Angioneogenese im Tumorbereich und Umfeld der Haut. Diese Tumoren wachsen dann auch nicht weiter.

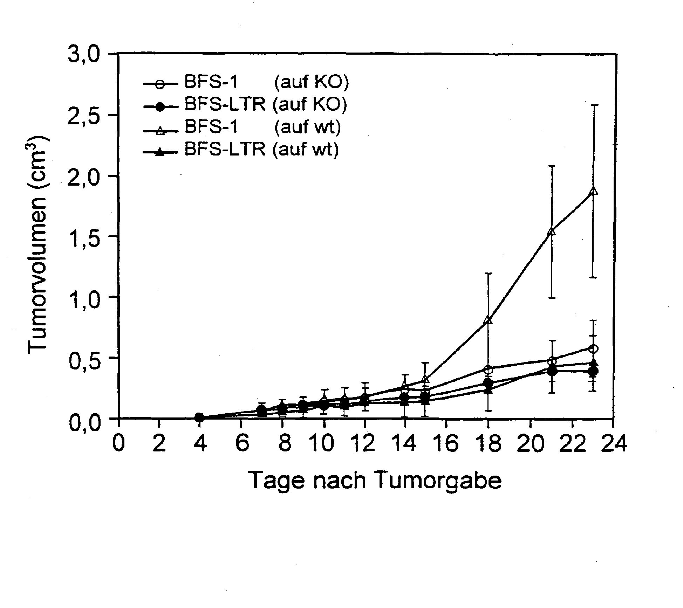

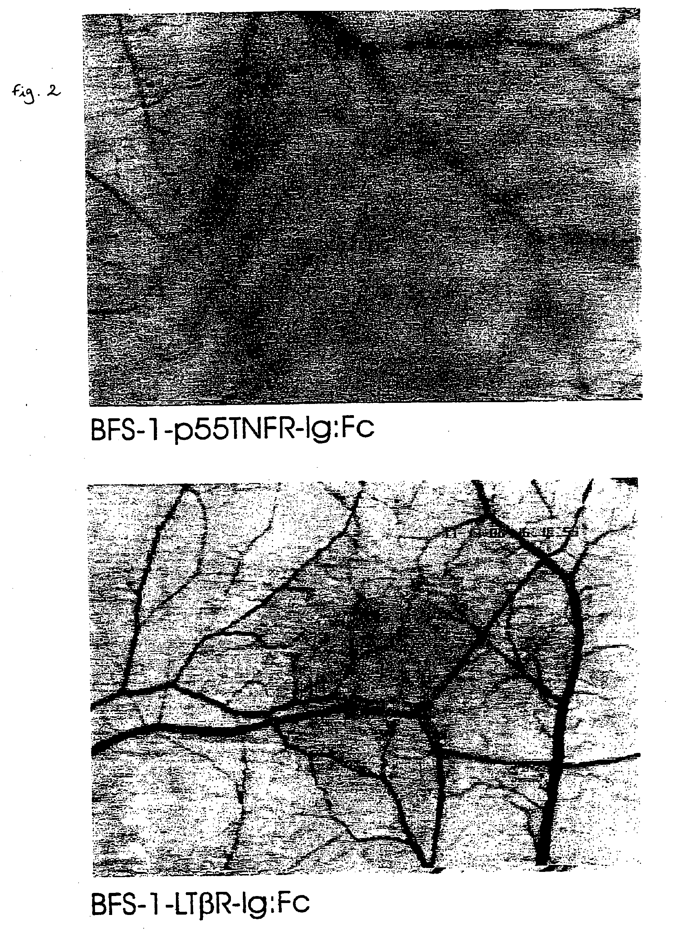

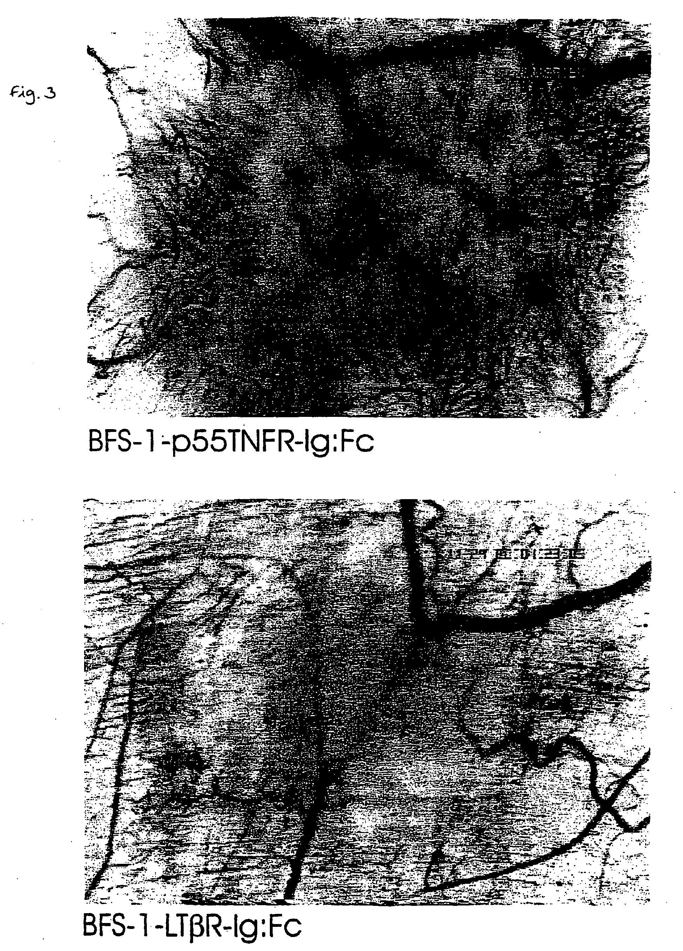

Auf den Abbildungen sind Aufnahmen der Kontrolltumoren (oben) und der LTβ-Rezeptor-Inhibitor transfizierten Tumoren (unten) am Tag 2 (Abb. 2) bzw. am Tag 9 (Abb. 3) nach Tumorzell-Applikation zu sehen.

## Patentansprüche

1. Arzneimittel zur Verhinderung von Tumorangiogenese umfassend ein Fusionsprotein aus Lymphotoxin β Rezeptor (LTBR) und Immunglobulin.

## Claims

1. Medicament for the prevention of tumor angiogenesis comprising a fusion protein of lymphotoxin β receptor (LTBR) and immunoglobulin.

## Revendications

1. Médicament pour la prévention de l'angiogénèse tumorale comprenant une protéine de fusion du récepteur de la lymphotoxine bêta (LTBR) et d'immunoglobuline.
